(19) Europäisches Patentamt
European Patent Office
Office européen des brevets

(11) **EP 1 636 161 B1**

(12) **EUROPÄISCHE PATENTSCHRIFT**

(45) Veröffentlichungstag und Bekanntmachung des Hinweises auf die Patenterteilung:
**17.01.2007 Patentblatt 2007/03**

(51) Int Cl.:
*C07C 51/265* [(2006.01)]   *C07C 63/16* [(2006.01)]

(21) Anmeldenummer: 04734261.3

(22) Anmeldetag: **21.05.2004**

(86) Internationale Anmeldenummer:
**PCT/EP2004/005510**

(87) Internationale Veröffentlichungsnummer:
**WO 2004/103943 (02.12.2004 Gazette 2004/49)**

(54) **HERSTELLUNG VON ALDEHYDEN, CARBONSÄUREN UND/ODER CARBONSÄUREANHYDRIDEN MITTELS VANADIUMOXID, TITANDIOXIDE UND ANTIMONOXID ENTHALTENDER KATALYSATOREN**

PRODUCTION OF ALDEHYDES, CARBOXYLIC ACIDS, AND/OR CARBOXYLIC ACID ANHYDRIDES BY MEANS OF CATALYSTS CONTAINING VANADIUM OXIDE, TITANIUM DIOXIDE, AND ANTIMONY OXIDE

PRODUCTION D'ALDEHYDES, D'ACIDES CARBOXYLIQUES ET/OU D'ANHYDRIDES D'ACIDES CARBOXYLIQUES A L'AIDE D'OXYDE DE VANADIUM, D'OXYDE DE TITANE ET DE CATALYSEURS CONTENANT DE L'OXYDE D'ANTIMOINE

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.05.2003 DE 10323461**

(43) Veröffentlichungstag der Anmeldung:
**22.03.2006 Patentblatt 2006/12**

(73) Patentinhaber: **BASF Aktiengesellschaft**
**67056 Ludwigshafen (DE)**

(72) Erfinder:
• **STORCK, Sebastian**
**68167 Mannheim (DE)**
• **ZÜHLKE, Jürgen**
**67346 Speyer (DE)**
• **NETO, Samuel**
**01099 Dresden (DE)**
• **ROSOWSKI, Frank**
**68165 Mannheim (DE)**

(74) Vertreter: **Reitstötter - Kinzebach Patentanwälte**
**Sternwartstrasse 4**
**81679 München (DE)**

(56) Entgegenhaltungen:
EP-A- 0 163 231       EP-A- 0 539 878
EP-A- 0 676 400

EP 1 636 161 B1

**Beschreibung**

Die Erfindung betrifft ein Verfahren zur Herstellung von Phthalsäureanhydrid, bei dem man einen gasförmigen Strom, der einen unter o-Xylol und/oder Naphthalin ausgewählten aromatischen Kohlenwasserstoff und ein molekularen Sauerstoff enthaltendes Gas umfasst, bei erhöhter Temperatur über ein Katalysatorbett leitet.

Eine Vielzahl von Aldehyden, Carbonsäuren und/oder Carbonsäureanhydriden wird technisch durch katalytische Gasphasenoxidation von aromatischen Kohlenwasserstoffen wie Benzol, o-, m- oder p-Xylol, Naphthalin, Toluol oder Durol (1,2,4,5-Tetramethylbenzol) in Festbettreaktoren, vorzugsweise Rohrbündelreaktoren, hergestellt. Dabei werden je nach Ausgangsmaterial beispielsweise Benzaldehyd, Benzoesäure, Maleinsäureanhydrid, Phthalsäureanhydrid, Isophthalsäure, Terephthalsäure oder Pyromellithsäureanhydrid gewonnen. Hierzu werden überwiegend Katalysatoren auf der Basis von Vanadiumoxid und Titandioxid verwendet.

Die Gasphasenoxidation ist stark exotherm. Es kommt zur Ausbildung lokaler Temperaturmaxima, so genannter Hot spots, in denen eine höhere Temperatur herrscht als im übrigen Teil der Katalysatorschüttung. Diese Hot spots geben Anlass zu Nebenreaktionen, wie der Totalverbrennung des Ausgangsmaterials, oder führen zur Bildung unerwünschter, vom Reaktionsprodukt nicht oder nur mit viel Aufwand abtrennbarer Nebenprodukte. Außerdem kann der Katalysator ab einer bestimmten Hot spot-Temperatur irreversibel geschädigt werden.

Zur Abschwächung dieser Hot spots wurde dazu übergegangen, unterschiedlich aktive Katalysatoren schichtweise in der Katalysatorschüttung anzuordnen, wobei der weniger aktive Katalysator zum Gaseintritt hin und der aktivere Katalysator zum Gasaustritt hin gelegen ist.

Die EP-A 1 063 222 offenbart ein Verfahren zur Herstellung von Phthalsäureanhydrid durch Gasphasenoxidation an drei oder mehr Katalysatorschichten. Es werden Katalysatoren ausgehend von einer Aufschlämmung hergestellt, die neben Titandioxid und untergeordneten Komponenten 121,86 g Ammoniummetavanadat und 37,89 g Antimontrioxid bzw. 96,48 g Ammoniummetavanadat und 37,50 g Antimontrioxid enthält. Die Aktivität der Katalysatoren wird durch Variation des Gehalts an Cäsium und Phosphor gesteuert.

Die US-A 4,356,112 beschreibt, dass bei Katalysatoren zur Herstellung von Phthalsäureanhydrid durch Mitverwendung von Antimon die Wärmebeständigkeit und Selektivität der Katalysatoren verbessert wird. Die besten Phthalsäureanhydrid-Ausbeuten werden bei einer Zweischichtanordnung mit einem Katalysator der Zusammensetzung $V_2O_5:TiO_2:Nb_2O_5:P_2O_5:Cs_2O:Sb_2O_3 = 3:97:0,5:0,2:0,4:2,5$ in der ersten Schicht und $V_2O_5:TiO_2:Nb_2O_5:P_2O_5:Cs_2O:Sb_2O_3 = 3:97:0,5:0,2:0,4:1,0$ in der zweiten Schicht erhalten.

Die DE 198 39 001 lehrt Schal,enkatalysatoren für die Gasphasenoxidation von Kohlenwasserstoffen, die Vanadiumoxid, Titandioxid und Antimonoxid enthalten und bei denen auf einem Träger zwei oder mehr Schichten katalytisch aktiver Massen aufgebracht sind, wobei die äußere Schicht einen verringerten Antimongehalt aufweist.

Die EP-A 0 522 871 beschreibt einen Katalysator zur Herstellung von Phthalsäureanhydrid durch Gasphasenoxidation, bei dessen Herstellung eine fünfwertige Antimonverbindung als Antimonquelle verwendet wird. Die offenbarten Katalysatoren zeigen $V_2O_5:Sb_2O_3$-Verhältnisse von 2:2,0 bzw. $V_2O_5:Sb_2O_5$-Verhältnisse von 2:2,5.

Die EP-A 539 878 offenbart die Herstellung von Phthalsäureanhydrid durch Gasphasenoxidation von o-Xylol und Naphthalin an einem ersten Katalysator, der Vanadiumoxid, Titandioxid, Nb, P, Sb und wenigstens eine unter K, Cs, Rb und TI ausgewählte Komponente enthält, und einem zweiten Katalysator, worin der Anteil dieser Komponente gegenüber dem ersten Katalysator reduziert ist.

Die EP-A 163 231 beschreibt einen Trägerkatalysator zur Herstellung von Pyromellitsäure(anhydrid), der $V_2O_5$; $TiO_2$ und/oder $SnO_2$ und/oder $ZrO_2$; eine Phosphor- und eine Niobverbindung sowie gegebenenfalls eine Antimonverbindung enthält.

Mit zunehmender Alterung des Katalysators in der ersten Schicht nimmt dessen Aktivität und damit sein Beitrag zum Gesamtumsatz der Reaktion ab. Es gelangt ein höherer Anteil nicht umgesetzter Kohlenwasserstoffe oder teiloxidierter Intermediate in die nachgelagerten Katalysatorschichten. Die Reaktion verlagert sich zunehmend in die nachgelagerten Katalysatorschichten, die zwar eine höhere Aktivität aber eine geringere Selektivität aufweisen. Insgesamt sinkt die Wertproduktausbeute daher mit zunehmender Betriebsdauer.

Der Erfindung liegt die Aufgabe zu Grunde, ein Verfahren bereitzustellen, das es gestattet, die gewünschten Oxidationsprodukte über längere Zeiträume in hoher Ausbeute zu erhalten.

Diese Aufgabe wird erfindungsgemäß gelöst durch ein Verfahren zur Herstellung von Phthalsäureanhydrid, bei dem man einen gasförmigen Strom, der einen unter o-Xylol und/oder Naphthalin ausgewählten aromatischen Kohlenwasserstoff und ein molekularen Sauerstoff enthaltendes Gas umfasst, bei erhöhter Temperatur über ein Bett eines ersten Katalysators und ein Bett eines in Strömungsrichtung des gasförmigen Stroms stromabwärts zum ersten Katalysator gelegenen zweiten Katalysators mit höherer Aktivität als der erste Katalysator leitet, wobei die katalytisch aktive Masse des ersten Katalysators zumindest Vanadiumoxid, Titandioxid und Antimonoxid enthält und das Verhältnis von Vanadium, berechnet als $V_2O_5$, zu Antimon, berechnet als $Sb_2O_3$, im ersten Katalysator 3,5:1 bis 4,5:1, vorzugsweise 3,8:1 bis 4,5:1, beträgt.

Es wurde gefunden, dass ein niedrigeres Verhältnis von Vanadium zu Antimon als das angegebene im ersten Katalysator zu einer raschen Alterung des Katalysators und zur Verlagerung der Reaktion in die nachgelagerten Katalysatorschichten führt. Ein höheres Verhältnis als das angegebene führt insbesondere bei hohen Kohlenwasserstoff-Beladungen des Gasstroms zu einer ungenügenden Gesamtausbeute.

Vorzugsweise enthält auch die katalytisch aktive Masse des zweiten Katalysators zumindest Vanadiumoxid, Titandioxid und Antimonoxid und insbesondere ist das Verhälnis von Vanadium, berechnet als $V_2O_5$, zu Antimon, berechnet als $Sb_2O_3$, im zweiten Katalysator kleiner oder gleich dem entsprechenden Verhältnis im ersten Katalysator.

Bevorzugt sind Verfahren, bei denen man den gasförmigen Strom außerdem über ein Bett eines stromabwärts zum zweiten Katalysator gelegenen dritten und gegebenenfalls vierten Katalysators leitet. In der Regel enthält auch die katalytisch aktive Masse des dritten und vierten Katalysators Vanadiumoxid und Titandioxid.

Die Aktivität der Katalysatoren nimmt in Strömungsrichtung des Gasstroms stufenweise zu. Maßnahmen zur Steuerung der Aktivität von Gasphasenoxidationskatalysatoren auf Basis von Vanadiumoxid und Titandioxid sind dem Fachmann an sich bekannt.

So können in der katalytisch aktiven Masse oxidische Verbindungen enthalten sein, die als Promotoren die Aktivität und Selektivität des Katalysators beeinflussen, beispielsweise indem sie seine Aktivität absenken oder erhöhen.

Als aktivitätssenkende Promotoren seien beispielhaft die Alkalimetalloxide, insbesondere Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt. In der Regel wird aus dieser Gruppe Cäsium als Promotor verwendet. Als Quellen dieser Elemente kommen die Oxide oder Hydroxide oder die thermisch in Oxide überführbare Salze wie Carboxylate, insbesondere die Acetate, Malonate oder Oxalate, Carbonate, Hydrogencarbonate oder Nitrate in Betracht.

Als aktivitätserhöhender Zusatz eignen sich vor allem oxidische Phosphorverbindungen insbesondere Phosphorpentoxid. Als Phosphorquelle kommen insbesondere Phosphorsäure, phosphorige Säure, hypophosphorige Säure, Ammoniumphosphat oder Phosphorsäureester und vor allem Ammoniumdihydrogenphosphat in Betracht

Eine weitere Möglichkeit der Aktivitätssteuerung besteht in der Variation des Anteils der Aktivmasse am Gesamtgewicht des Katalysators, wobei höhere Aktivmassengehalte eine höhere Aktivität bedingen und umgekehrt.

Üblicherweise wird das Titandioxid in der Anatas-Form verwendet. Seine BET-Oberfläche beträgt im Allgemeinen 5 bis 50 $m^2$/g, vorzugsweise 8 bis 28 $m^2$/g. Bevorzugt besteht das eingesetzte Titandioxid in mindestens einer Katalysatorschicht aus einem Gemisch von Titandioxiden unterschiedlicher BET-Oberflächen. Dieses Gemisch aus Titandioxid Typen beinhaltet beispielsweise ein niederoberflächiges Titandioxid mit einer BET-Oberfläche von vorteilhaft 5 bis 15 $m^2$/g, insbesondere 5 bis 10, und ein höheroberflächiges Titandioxid mit einer BET-Oberfläche von vorteilhaft 10 bis 70 $m^2$/g, insbesondere 15 bis 50. Insbesondere besteht das eingesetzte Titandioxid aus den zwei genannten Titandioxid Typen. Derartige Mischungen mit niederoberflächigem $TiO_2$ haben den Vorteil, dass sich die BET-Oberfläche über die Lebensdauer des Katalysators nicht verändert. Somit wird eine hohe Stabilität der Aktivität, d.h. eine längere Lebensdauer des Katalysators gewährleistet.

Als Vanadiumquelle eignen sich besonders Vanadiumpentoxid oder Ammoniummetavanadat.

Als Antimonquelle eignen sich verschiedene Antimonoxide, insbesondere Antimontrioxid. Im Allgemeinen verwendet man Antimontrioxid mit einer mittleren Teilchengröße (Maximum der Teilchengrößenverteilung) von 0,1 bis 10 μm. Besonders bevorzugt verwendet man als Quelle des Antimonoxids im ersten Katalysator teilchenförmiges Antimontrioxid mit einer mittleren Teilchengröße von 0,5 bis 5 μm, insbesondere 1 bis 4 μm. Die Verwendung eines Antimontrioxids der angegebenen Teilchengröße führt zu einer deutlichen Verbesserung der Aktivität und Selektivität des ersten Katalysators.

Bei den im erfindungsgemäßen Verfahren verwendeten Katalysatoren handelt es sich im Allgemeinen um Schalenkatalysatoren, bei denen die katalytisch aktive Masse schalenförmig auf einem inerten Träger aufgebracht ist. Die Schichtdicke der katalytisch aktiven Masse beträgt in der Regel 0,02 bis 0,2 mm, vorzugsweise 0,05 bis 0,1 mm. Im Allgemeinen weisen die Katalysatoren eine schalenförmig aufgebrachte Aktivmasseschicht mit im Wesentlichen homogener chemischer Zusammensetzung auf.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz ($SiO_2$), Porzellan, Magnesiumoxid, Zinndioxid, Siliciumcarbid, Rutil, Tonerde ($Al_2O_3$), Aluminiumsilikat, Steatit (Magnesiumsilikat), Zirkoniumsilikat, Cersilikat oder Mischungen dieser Trägermaterialien. Das Trägermaterial ist in der Regel nichtporös. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 4 bis 7 mm verwendet.

Die Aufbringung der einzelnen Schichten des Schalenkatalysators kann mit beliebigen an sich bekannten Methoden erfolgen, z. B. durch Aufsprühen von Lösungen oder Suspensionen in der Dragiertrommel oder Beschichtung mit einer Lösung oder Suspension in einer Wirbelschicht. Dabei können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei mit Vorteil Bindermengen von 3 bis 20 Gew.-%, bezogen auf den Feststoffgehalt der Lösung der Aktivmassenbestandteile, eingesetzt werden. Wird die katalytisch aktive Masse ohne organische Bindemittel auf den Träger aufgetragen, so sind Beschichtungstemperaturen über 150 °C von Vorteil. Bei Zusatz der oben angegebenen Bindemittel liegen die brauchbaren Beschichtungstemperaturen je nach verwendetem Bindemittel zwischen 50 und 450 °C. Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

In einer bevorzugten Ausführungsform des erfindungsgemäßen Verfahrens mit drei Katalysatorschichten weisen die Katalysatoren folgende Zusammensetzung auf (wobei die erste Schicht die in Strömungsrichtung des Gasstroms am weitesten stromaufwärts angeordnete Schicht ist):

für die erste Schicht:

7 bis 10 Gew.-% Aktivmasse bezogen auf den gesamten Katalysator, wobei diese Aktivmasse:
6 bis 11 Gew.-% Vanadiumpentoxid
1,2 bis 3 Gew.-% Antimontrioxid
0,1 bis 1 Gew.-% eines Alkali (berechnet als Alkalimetall), insbesondere Cäsiumoxid

enthält und als Rest auf 100 Gew.-% Titandioxid, vorzugsweise in Anatasmodifikation mit einer BET-Oberfläche von 5 bis 30 m$^2$/g

für die zweite Schicht:

7 bis 12 Gew.-% Aktivmasse bezogen auf den gesamten Katalysator, wobei diese Aktivmasse:
5 bis 13 Gew.-% Vanadiumpentoxid
0 bis 3 Gew.-% Antimontrioxid
0 bis 0,4 Gew.-% eines Alkali (berechnet als Alkalimetall), insbesondere Cäsiumoxid
0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 10 bis 40 m$^2$/g

für die dritte Schicht:

8 bis 12 Gew.-% Aktivmasse bezogen auf den gesamten Katalysator, wobei diese Aktivmasse:
5 bis 30 Gew.-% Vanadiumpentoxid
0 bis 3 Gew.-% Antimontrioxid
0 bis 0,3 Gew.-% eines Alkali (berechnet als Alkalimetall), insbesondere Cäsiumoxid
0,05 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 50 m$^2$/g.

Das Verhältnis der von der ersten, zweiten und dritten Schicht eingenommenen Volumina beträgt vorzugsweise 120 bis 200 : 50 bis 100 : 50 bis 100.

In einer bevorzugten Ausführungsform des erfindungsgemäßen Verfahrens mit vier Katalysatorschichten weisen die Katalysatoren folgende Zusammensetzung auf (wobei die erste Schicht die in Strömungsrichtung des Gasstroms am weitesten stromaufwärts angeordnete Schicht ist):

für die erste Schicht:

7 bis 10 Gew.-% Aktivmasse bezogen auf den gesamten Katalysator, wobei diese Aktivmasse:
6 bis 11 Gew.-% Vanadiumpentoxid

1,2 bis 3 Gew.-% Antimontrioxid

0,1 bis 1 Gew.-% eines Alkali (berechnet als Alkalimetall), insbesondere Cäsiumoxid

enthält und als Rest zu 100 Gew.-% Titandioxid, vorzugsweise in Anatasmodifikation mit einer BET-Oberfläche von 5 bis 30 m$^2$/g

für die zweite Schicht:

7 bis 10 Gew.-% Aktivmasse bezogen auf den gesamten Katalysator, wobei diese Aktivmasse:

4 bis 15 Gew.-% Vanadiumpentoxid

0 bis 3 Gew.-% Antimontrioxid

0,1 bis 1 Gew.-% eines Alkali (berechnet als Alkalimetall), insbesondere Cäsiumoxid

0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 10 bis 35 m$^2$/g

für die dritte Schicht:

7 bis 10 Gew.-% Aktivmasse bezogen auf den gesamten Katalysator, wobei diese Aktivmasse:

5 bis 13 Gew.-% Vanadiumpentoxid

0 bis 3 Gew.-% Antimontrioxid

0 bis 0,4 Gew.-% eines Alkali (berechnet als Alkalimetall), insbesondere Cäsiumoxid

0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 40 m$^2$/g

für die vierte Schicht:

8 bis 12 Gew.-% Aktivmasse bezogen auf den gesamten Katalysator, wobei diese Aktivmasse:

10 bis 30 Gew.-% Vanadiumpentoxid

0 bis 3 Gew.-% Antimontrioxid

0,05 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 50 m$^2$/g.

Das Verhältnis der von der ersten, zweiten, dritten und vierten Schicht einegenommenen Volumina beträgt vorzugsweise 80 bis 160 : 20 bis 60 : 30 bis 100 : 40 bis 90.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 A beschrieben ist. Als Katalysator verwendet man dabei im Vergleich zum Katalysator der letzten Schicht vorzugsweise einen noch aktiveren Katalysator.

Das erfindungsgemäße Verfahren eignet sich zur Herstellung von Phthalsäureanhydrid aus o-Xylol und/oder Naphthalin.

Zu diesem Zweck werden die Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h$^{-1}$ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im allgemeinen mit 30 g bis 150 g je Nm$^3$ Gas, vorzugsweise 60 bis 120 g je Nm$^3$, des zu oxidierenden, aromatischen Kohlenwasserstoffs beschickt.

Man kann zwei oder mehr Zonen, vorzugsweise zwei Zonen der im Reaktionsrohr befindlichen Katalysatorschüttung auf unterschiedliche Reaktionstemperaturen thermostatisieren, wozu beispielsweise Reaktoren mit getrennten Salzbädern eingesetzt werden können. Alternativ kann die Gasphasenoxidation auch ohne Aufteilung in Temperaturzonen bei einer Reaktionstemperatur durchgeführt werden.

Die Erfindung wird durch die beigefügte Figur und die folgenden Beispiele näher veranschaulicht. In den Beispielen ist die in Strömungsrichtung des Gasstroms am weitesten stromaufwärts gelegene Katalysatorschicht als Oberschicht, die am weitesten stromabwärts gelegene Katalysatorschicht als Unterschicht bezeichnet. Dazwischen befinden sich eine oder zwei Mittelschichten. Die Messung der Teilchengröße des Antimontrioxids erfolgte mit Hilfe eines Fritsch Particle Sizer "Analysette 22" im Messbereich von 0,3 bis 300 $\mu$m mit einer Auflösung von 62 Kanälen. Die Messdauer betrug 2 Scans. Die Auswertung erfolgte nach der Frauenhofer-Methode.

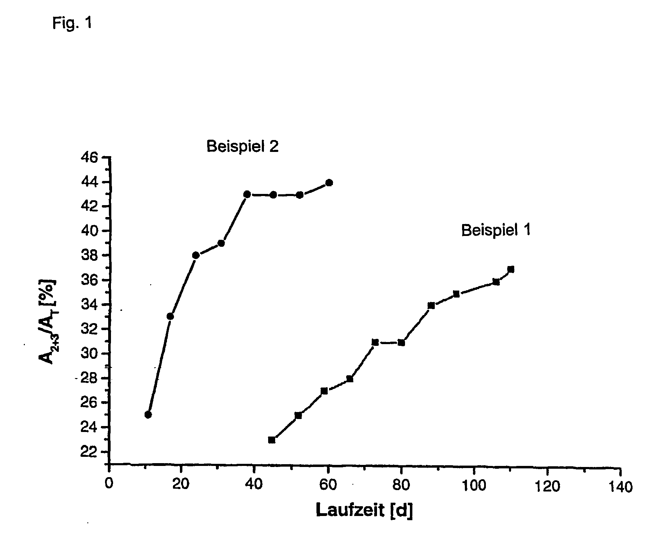

Fig. 1 zeigt den zeitlichen Verlauf der Stabilitätskennzahl, die die in den der ersten Schicht nachgeordneten Katalysatorschichten erzeugte Wärmemenge relativ zur Gesamtwärmemenge angibt, für die Katalysatoren der Beispiele 1 und 2.

Beispiele

Herstellungsbeispiel 1 (erfindungsgemäß):

Oberschicht:

29,8 g Anatas (BET-Oberfläche 9 m$^2$/g), 69,6 g Anatas (BET-Oberfläche 20 m$^2$/g), 7,1 g $V_2O_5$, 1,8 g $Sb_2O_3$ (Maximum der Partikelgrößenverteilung von 2,36 $\mu$m), 0,46 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, Außendurchmesser x Länge x Innendurchmesser, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 6,8 Gew.-% $V_2O_5$, 1,7 Gew.-% $Sb_2O_3$, 0,33 Gew.-% Cs. (Verhältnis $V_2O_5$:$Sb_2O_3$ = 4).

Mittelschicht:

26,6 g Anatas (BET-Oberfläche 9 m$^2$/g), 79,9 g Anatas (BET-Oberfläche 20 m$^2$/g), 7,55 g $V_2O_5$, 1,89 g $Sb_2O_3$, 0,14 g $Cs_2CO_3$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,7 % des Gesamtgewichts des fertigen Katalysators.

Die auf diese Weise aufgebrachte katalytisch aktive Masse enthielt nach Kalzination bei 400 °C für 4 h 6,4 Gew.-% $V_2O_5$, 1,7 Gew.-% $Sb_2O_3$, 0,09 Gew.-% Cs.

Unterschicht:

17,4 g Anatas (BET-OF 9 m$^2$/g), 69,6 g Anatas (BET-OF 27 m$^2$/g), 21,7 g $V_2O_5$, 1,5 g $NH_4H_2PO_4$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 9,0 % des Gesamtgewichts des fertigen Katalysators.

Die auf diese Weise aufgebrachte katalytisch aktive Masse enthielt nach Kalzination bei 400 °C für 4 h 20,0 Gew.-% $V_2O_5$, 0,37 Gew.-% P.

Beispiel 2 (Vergleich):

Oberschicht:

29,8 g Anatas (BET-Oberfläche 9 m$^2$/g), 69,6 g Anatas (BET-Oberfläche 20 m$^2$/g), 7,1 g $V_2O_5$, 2,3 g $Sb_2O_3$ (Maximum der Partikelgrößenverteilung von 2,36 $\mu$m), 0,46 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 6,8 Gew.-% $V_2O_5$, 2,2 Gew.-% $Sb_2O_3$, 0,33 Gew.-% Cs (Verhältnis $V_2O_5$:$Sb_2O_3$ = 3).

Mittelschicht:

26,6 g Anatas (BET-Oberfläche 9 $m^2$/g), 79,9 g Anatas (BET-OF 20 $m^2$/g), 7,55 g $V_2O_5$, 1,89 g $Sb_2O_3$, 0,14 g $Cs_2CO_3$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,7 % des Gesamtgewichts des fertigen Katalysators.
Die auf diese Weise aufgebrachte katalytisch aktive Masse enthielt nach Kalzination bei 400 °C für 4 h 6,4 Gew.-% $V_2O_5$, 1,7 Gew.-% $Sb_2O_3$, 0,09 Gew.-% Cs.

Unterschicht:

17,4 g Anatas (BET-Oberfläche 9 $m^2$/g), 69,6 g Anatas (BET-Oberfläche 27 $m^2$/g), 21,7 g $V_2O_5$, 1,5 g $NH_4H_2PO_4$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 9,0 % des Gesamtgewichts des fertigen Katalysators.
Die auf diese Weise aufgebrachte katalytisch aktive Masse enthielt nach Kalzination bei 400 °C für 4 h 20,0 Gew.-% $V_2O_5$, 0,37 Gew.-% P.

Herstellungsbeispiel 3 (erfindungsgemäß):

Oberschicht:

29,3 g Anatas (BET-Oberfläche 9 $m^2$/g), 69,8 g Anatas (BET-Oberfläche 20 $m^2$/g), 7,8 g $V_2O_5$, 1,9 g $Sb_2O_3$ (Maximum der Partikelgrößenverteilung von 2,36 µm), 0,49 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.
Die auf diese Weise aufgebrachte katalytisch aktive Masse enthielt nach Kalzination bei 400 °C für 4 h 7,1 Gew.-% $V_2O_5$, 1,8 Gew.-% $Sb_2O_3$, 0,36 Gew.-% Cs (Verhältnis $V_2O_5$:$Sb_2O_3$ = 4).

Mittelschicht 1:

24,6 g Anatas (BET-Oberfläche 9 $m^2$/g), 74,5 g Anatas (BET-Oberfläche 27 $m^2$/g), 7,8 g $V_2O_5$, 2,6 g $Sb_2O_3$, 0,35 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.
Die auf diese Weise aufgebrachte katalytisch aktive Masse enthielt nach Kalzination bei 400°C für 4 h 7,1 Gew.-% $V_2O_5$, 2,4 Gew.-% $Sb_2O_3$, 0,26 Gew.-% Cs.

Mittelschicht 2:

24,8 g Anatas (BET-Oberfläche 9 $m^2$/g), 74,5 g Anatas (BET-Oberfläche 27 $m^2$/g), 7,8 g $V_2O_5$ 2,6 g $Sb_2O_3$, 0,13 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,10 Gew.-% Cs.

Unterschicht:

17,2 g Anatas (BET-Oberfläche 9 m$^2$/g), 69,1 g Anatas (BET-Oberfläche 27 m$^2$/g), 21,9 g $V_2O_5$, 1,5 g $NH_4H_2PO_4$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 20,0 Gew.-% $V_2O_5$, 0,38 Gew.-% P.

Herstellungsbeispiel 4 (Vergleich):

Oberschicht:

29,3 g Anatas (BET-Oberfläche 9 m$^2$/g), 69,8 g Anatas (BET-Oberfläche 20 m$^2$/g), 7,8 g $V_2O_5$, 2,6 g $Sb_2O_3$, (Maximum der Partikelgrößenverteilung von 2,36 μm), 0,49 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,36 Gew.-% Cs (Verhältnis $V_2O_5$:$Sb_2O_3$ = 3).

Mittelschicht 1:

24,6 g Anatas (BET-Oberfläche 9 m$^2$/g), 74,5 g Anatas (BET-Oberfläche 27 m$^2$/g), 7,8 g $V_2O_5$, 2,6 g $Sb_2O_3$, 0,35 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,26 Gew.-% Cs.

Mittelschicht 2:

24,8 g Anatas (BET-Oberfläche 9 m$^2$/g), 74,5 g Anatas (BET-Oberfläche 27 m$^2$/g), 7,8 g $V_2O_5$, 2,6 g $Sb_2O_3$, 0,13 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,10 Gew.-% Cs.

Unterschicht:

17,2 g Anatas (BET-Oberfläche 9 m$^2$/g), 69,1 g Anatas (BET-Oberfläche 27 m$^2$/g), 21,9 g $V_2O_5$, 1,5 g $NH_4H_2PO_4$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 20,0 Gew.-% $V_2O_5$, 0,38 Gew.-% P.

Herstellungsbeispiel 5:

Oberschicht:

29,3 g Anatas (BET-Oberfläche 9 m$^2$/g), 69,8 g Anatas (BET-Oberfläche 20 m$^2$/g), 7,8 g $V_2O_5$, 1,9 g $Sb_2O_3$ (Maximum der Partikelgrößenverteilung von 7,42 $\mu$m), 0,49 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 1,8 Gew.-% $Sb_2O_3$, 0,36 Gew.-% Cs.

Mittelschicht 1:

24,6 g Anatas (BET-Oberfläche 9 m$^2$/g), 74,5 g Anatas (BET-Oberfläche 27 m$^2$/g), 7,8 g $V_2O_5$, 2,6 g $Sb_2O_3$, 0,35 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,26 Gew.-% Cs.

Mittelschicht 2:

24,8 g Anatas (BET-Oberfläche 9 m$^2$/g), 74,5 g Anatas (BET-Oberfläche 27 m$^2$/g), 7,8 g $V_2O_5$, 2,6 g $Sb_2O_3$, 0,13 g $Cs_2CO_3$ wurden in 550 ml entionisiertem Wasser suspendiert und 15 h lang gerührt. Dieser Suspension wurden anschließend 50 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 7,1 Gew.-% $V_2O_5$, 2,4 Gew.-% $Sb_2O_3$, 0,10 Gew.-% Cs.

Unterschicht:

17,2 g Anatas (BET-Oberfläche 9 m$^2$/g), 69,1 g Anatas (BET-Oberfläche 27 m$^2$/g), 21,9 g $V_2O_5$, 1,5 g $NH_4H_2PO_4$ wurden in 550 ml entionisiertem Wasser suspendiert und 15 h lang gerührt. Dieser Suspension wurden anschließend 55 g einer wässrigen Dispersion (50 Gew.-%) aus Vinylacetat und Vinyllaurat zugegeben. Anschließend erfolgte das Aufbringen der Suspension auf 1200 g Steatitformkörper (Magnesiumsilikat) in Form von Ringen (7 x 7 x 4 mm, AD x L x ID) durch Aufsprühen. Das Gewicht der aufgetragenen Aktivmassenschale betrug 8,0 % des Gesamtgewichts des fertigen Katalysators.

Die auf diese Weise aufgebrachte katalytisch aktive Masse enthielt nach Kalzination bei 400 °C für 4 h 20,0 Gew.-% $V_2O_5$, 0,38 Gew.-% P.

Katalytische Tests:

Die Tests erfolgten in einem salzbadgekühlten Reaktor mit einer Länge von 3,85 m und einem Innendurchmesser von 25 mm, in den die Katalysatoren der Beispiele 1 bis 5 beginnend mit dem Unterschichtkatalysator eingefüllt wurden. Zur Aufnahme eines Temperaturprofils war der Reaktor mit einem über die gesamte Reaktorlänge beweglichen Thermoelement ausgestattet. Das Element wurde in einer Hülse mit einem Außendurchmesser von 2 mm gehalten. Durch das Rohr wurden stündlich von oben nach unten 4,0 Nm$^3$-Luft mit o-Xylol (mindestens 98,5 % Reinheit) von 80

oder 100 g/Nm$^3$ geleitet. Dabei wurden die in der nachstehenden Tabelle zusammengefassten Ergebnisse erhalten ("PSA-Ausbeute" bedeutet Masseteile erhaltenes Phthalsäureanhydrid, bezogen auf 100 Masseteile reines o-Xylol).

Bestimmung der Stabilitätskennzahl

Die Stabilitätskennzahl S(Stab) gibt die in den der ersten Schicht nachgeordneten Katalysatorschichten erzeugte Wärmemenge relativ zur Gesamtwärmemenge an. Sie ist durch folgende Gleichung definiert:

$$S(Stab) = A(2 + 3 + ...)/A(1 + 2 + 3 + ....)$$

worin A(1 + 2 + 3 + ....) für die integrierte Fläche unter der Temperatur-Schütthöhen-Kurve aller Katalysatorschichten steht und A(2 + 3 + ....) für die entsprechende Fläche für die der ersten Schicht nachgeordneten Katalysatorschichten. Die Temperatur-Schütthöhen-Kurve kann leicht durch grafische Auftragung der mittels des Termoelements bestimmten Temperatur gegen die Position des Thermoelements erhalten werden.

Fig. 1 zeigt den zeitlichen Verlauf von S(Stab) für die Katalysatoren der Beispiele 1 und 2 im Vergleich. Man sieht, dass beim erfindungsgemäßen Beispiel 1 im betrachteten Zeitraum geringere Wärmemengen in der zweiten und dritten Katalysatorschicht entstehen.

Tabelle 1:

|  | Beispiel 1 | Beispiel 2 | Beispiel 3 | Beispiel 4 | Beispiel 5 |
|---|---|---|---|---|---|
| **Schüttungslänge [cm]** | 170, 70, 70 | 170, 70, 70 | 130, 50, 80, 60 | 130, 50, 80, 60 | 130, 50, 80, 60 |
| **Beladung [g/Nm$^3$]** | 80 | 80 | 100 | 100 | 100 |
| **Laufzeit [d]** | 60 | 60 | 43 | 44 | 42 |
| **Salzbadtemp. [°C]** | 360 | 361 | 354 | 353 | 350 |
| **Hotspot-Temp. OS [°C]** | 440 | 435 | 440 | 437 | 444 |
| **Phthalid [Gew.-%]** | 0,01 | 0,04 | 0,02 | 0,04 | 0,07 |
| **PSA-Ausbeute** | 114,2 | 112,4 | 113,5 | 113,0 | 112,7 |

Der Vergleich von Beispiel 1 und 2 bzw. Beispiel 3 und 4 zeigt, dass unter Verwendung eines Katalysators mit erfindungsgemäßem V$_2$O$_5$:Sb$_2$O$_3$-Verhältnis in der ersten Schicht höhere Ausbeuten erzielt werden. Der Vergleich von Beispiel 3 und 5 zeigt den Einfluss der Teilchengröße des zur Herstellung des ersten Katalysators verwendeten Antimontrioxids.

**Patentansprüche**

1.  Verfahren zur Herstellung von Phthalsäureanhydrid, bei dem man einen gasförmigen Strom, der einen unter o-Xylol und/oder Naphthalin ausgewählten aromatischen Kohlenwasserstoff und ein molekularen Sauerstoff enthaltendes Gas umfasst, bei erhöhter Temperatur über ein Bett eines ersten Katalysators und ein Bett eines in Strömungsrichtung des gasförmigen Stroms stromabwärts zum ersten Katalysator gelegenen zweiten Katalysators mit höherer Aktivität als der erste Katalysator leitet, wobei die katalytisch aktive Masse des ersten Katalysators zumindest Vanadiumoxid, Titandioxid und Antimonoxid enthält und das Verhältnis von Vanadium, berechnet als V$_2$O$_5$, zu Antimon, berechnet als Sb$_2$O$_3$, im ersten Katalysator 3,5:1 bis 4,5:1 beträgt.

2.  Verfahren nach Anspruch 1, wobei das Verhältnis von Vanadium zu Antimon im ersten Katalysator 3,8:1 bis 4,5:1 beträgt.

3.  Verfahren nach Anspruch 1 oder 2, bei dem man als Quelle des Antimonoxids im ersten Katalysator teilchenförmiges Antimontrioxid mit einer mittleren Teilchengröße von 0,5 bis 5 μm verwendet.

4.  Verfahren nach einem der vorhergehenden Ansprüche, bei dem man den gasförmigen Strom außerdem über ein Bett eines stromabwärts zum zweiten Katalysator gelegenen dritten und gegebenenfalls vierten Katalysators leitet.

**5.** Verfahren nach einem der vorhergehenden Ansprüche, bei dem die katalytisch aktive Masse des zweiten Katalysators zumindest Vanadiumoxid, Titandioxid und Antimonoxid umfasst und das Verhältnis von Vanadium zu Antimon im zweiten Katalysator kleiner oder gleich dem entsprechenden Verhältnis im ersten Katalysator ist.

**6.** Verfahren nach einem der vorhergehenden Ansprüche, wobei die Beladung des gasförmigen 30 g bis 150 g aromatischer Kohlenwasserstoff pro Nm$^3$ Gas beträgt.

**Claims**

**1.** A process for preparing phthalic anhydride, in which a gaseous stream comprising an aromatic hydrocarbon, selected from o-xylene and/or naphthalene and a gas comprising molecular oxygen is passed at elevated temperature over a bed of a first catalyst and a bed which is made up of a second catalyst having a higher activity than the first catalyst and is located downstream of the first catalyst in the flow direction of the gaseous stream, wherein the catalytically active composition of the first catalyst comprises at least vanadium oxide, titanium dioxide and antimony oxide and the ratio of vanadium, calculated as $V_2O_5$, to antimony, calculated as $Sb_2O_3$, in the first catalyst is from 3.5:1 to 5:1.

**2.** The process according to claim 1, wherein the ratio of vanadium to antimony in the first catalyst is from 3.8:1 to 4.5:1.

**3.** The process according to claim 1 or 2, wherein the source of antimony oxide used for the first catalyst is particulate antimony trioxide having a mean particle size of from 0.5 to 5 $\mu$m.

**4.** The process according to any of the preceding claims, wherein the gaseous stream is additionally passed over a bed of a third and, if appropriate, fourth catalyst located downstream of the second catalyst.

**5.** The process according to any of the preceding claims, wherein the catalytically active composition of the second catalyst comprises at least vanadium oxide, titanium dioxide and antimony oxide and the ratio of vanadium to antimony in the second catalyst is less than or equal to the corresponding ratio in the first catalyst.

**6.** The process according to any of the preceding claims, wherein the loading of the gaseous aromatic hydrocarbon is from 30 to 150 g per standard m$^3$ of gas.

**Revendications**

**1.** Procédé de fabrication d'anhydride d'acide phtalique, dans lequel on achemine un courant gazeux, qui comprend un hydrocarbure aromatique choisi parmi le o-xylène et/ou le naphtalène et un gaz contenant de l'oxygène moléculaire, à température élevée sur un lit d'un premier catalyseur et sur un lit d'un deuxième catalyseur situé en aval du premier catalyseur dans le sens d'écoulement du courant gazeux et qui a une activité plus élevée que le premier catalyseur, la masse catalytiquement active du premier catalyseur contenant au moins de l'oxyde de vanadium, du dioxyde de titane et de l'oxyde d'antimoine et le rapport du vanadium, calculé sous la forme de $V_2O_5$, à l'antimoine, calculé sous la forme de $Sb_2O_3$, dans le premier catalyseur étant de 3,5:1 à 4,5:1.

**2.** Procédé selon la revendication 1, dans lequel le rapport du vanadium à l'antimoine dans le premier catalyseur est de 3,8:1 à 4,5:1.

**3.** Procédé selon la revendication 1 ou 2, dans lequel on utilise comme source d'oxyde d'antimoine dans le premier catalyseur du trioxyde d'antimoine particulaire d'une taille particulaire moyenne de 0,5 à 5 $\mu$m.

**4.** Procédé selon l'une quelconque des revendications précédentes, dans lequel on achemine le courant gazeux en outre sur un lit d'un troisième et éventuellement d'un quatrième catalyseur situé(s) en aval du deuxième catalyseur.

**5.** Procédé selon l'une quelconque des revendications précédentes, dans lequel la masse catalytiquement active du deuxième catalyseur comprend au moins de l'oxyde de vanadium, du dioxyde de titane et de l'oxyde d'antimoine et dans lequel le rapport du vanadium à l'antimoine dans le deuxième catalyseur est inférieur ou égal au rapport correspondant dans le premier catalyseur.

**6.** Procédé selon l'une quelconque des revendications précédentes, dans lequel le chargement de l'hydrocarbure aromatique gazeux représente 30 g à 150 g d'hydrocarbure aromatique par $Nm^3$ de gaz.

Fig. 1